# EUROPÄISCHE PATENTANMELDUNG

(11) **EP 3 482 744 A1**
(43) Veröffentlichungstag der Anmeldung: **15.05.2019**
(21) Anmeldenummer: 17201651.1
(22) Anmeldetag: 14.11.2017
(51) Int. Cl.: A61K 9/00, A61Q 7/00, A61K 45/06, A61K 47/10, A61K 47/18, A61K 9/06, A61K 9/107, A61K 31/085, A61K 31/14, A61K 31/192, A61K 31/196, A61K 31/4174, A61K 31/496, A61K 31/665, A61K 31/7056

(54) **EMULSIONEN ZUR TOPISCHEN BEHANDLUNG UROGENITALER UND DERMALER INFEKTIONEN**

(71) Anmelder: ProFem GmbH, 1180 Wien (AT)
(72) Erfinder: NOE, Marion, 1180 Wien (AT); NOE, Christian, 1180 Wien (AT)
(74) Vertreter: Sonn & Partner Patentanwälte

(57) **Zusammenfassung**

Emulsionen zur topischen Behandlung urogenitaler und dermaler Infektionen Die Erfindung betrifft eine Emulsion, die dadurch gekennzeichnet ist, dass ein antibakterieller Wirkstoff und ein antiadhäsiver Wirkstoff, vorzugsweise ein NSAID, kombiniert eingesetzt werden

## Beschreibung

Der Mensch lebt in Symbiose mit einer gigantischen Zahl von Mikroorganismen, welche vor allem seinen Darm und seine Hautoberfläche als Kommensalen besiedeln. Die Gesamtheit dieser Mikroorganismen wird als menschliches Mikrobiom bezeichnet. Eine ganz spezielle Zusammensetzung besitzt das Mikrobiom der Scheide der Frau, in welchem Milchsäurebakterien vorherrschen. Diese erzeugen in der Scheide unter normalen physiologischen Bedingungen ein schwach saures Milieu, das protektiv gegen bakterielle Infektionen wirkt. Dennoch treten aus verschiedenen Ursachen häufig Vaginalinfektionen auf, verursacht einerseits etwa durch die fast ubiquitär vorhandenen Pilze der Spezies Candida, welche sich leicht von Kommensalen zu Pathogenen wandeln, andererseits durch Bakterien wie Gardnerella vaginalis, Mobiluncus oder Prevotella spp., sowie durch Streptokokken oder Staphylokokken oder etwa durch typische Darmkeime, wie Enterobacter, E. coli und/oder etwa Klebsiella pneumoniae , welche alleine schon wegen der räumlichen Nähe der Körperöffnungen leicht durch Schmierinfektion in die Scheide gelangen können. Zunächst sind asymptomatische Fehlbesiedelungen die Folge. Wenn bestimmte Schwellenwerte an Fremdkeimen überschritten sind und infektionsunterstützende Faktoren hinzutreten, bricht letztlich ein virulentes infektiöses Geschehen aus. Wenn dieses nicht rechtzeitig behandelt wird, kann es in der Folge meist unter Biofilmbildung zur Chronifizierung der Infektion kommen. Schon asymptomatische Fehlbesiedelungen werden zunehmend auch als Risikofaktoren für Unfruchtbarkeit, Fehlgeburtlichkeit und Frühgeburtlichkeit aber auch bei Blasenentzündungen und bestimmten Formen der Inkontinenz erkannt.

Gegenstand der vorliegenden Erfindung sind Arzneimittel zur topischen Behandlung von bakteriellen Dysbiosen und manifesten Infektionskrankheiten, einschließlich von Mischinfektionen mit Pilzen und anderen Mikroorganismen. Demgemäß betrifft die vorliegende Erfindung eine Emulsion zur topischen Behandlung von dermalen und urogenitalen Infektionskrankheiten, insbesondere zur Anwendung in der topischen Behandlung von urogenitalen bakteriellen Infektionen, die dadurch gekennzeichnet ist, dass ein antibakterieller Wirkstoff und ein antiadhäsiver Wirkstoff, vorzugsweise ein NSAID, kombiniert eingesetzt werden.

Bakterielle Fehlbesiedelungen der Scheide bis hin zu symptomatischen Vaginalinfektionen sind häufig. Der Übergang von der asymptomatischen noch nicht entzündlichen Fehlbesiedelung zur symptomatischen Erkrankung wird durch die Begriffe Vaginose und Vaginitis näher bestimmt. Die Bezeichnungen aerobe Vaginose/Vaginitis und anaerobe Vaginose/Vaginitis (syn.: bakterielle Vaginose) stellen eine genauere Bestimmung der ursächlichen Erreger dar. Ein Problem, das allen vaginalen Infektionen, unabhängig vom Erregerorganismus gemeinsam ist, stellt die Ausbildung sogenannter Biofilme dar. Die Schleimhautoberflächen der betroffenen Organe (Vagina, Harnröhre, Blase, Penis) werden durch diese Biofilme, überzogen. In diesen sind die Mikroorganismen von einer Schicht aus Schleimsubstanzen umhüllt. Die einzelnen mikrobiellen Bestandteile des Biofilms übernehmen unterschiedliche Aufgaben im Zellverbund und tauschen auch Resistenzmechanismen aus. Die am häufigsten bei bakteriellen Dysvaginosen aufzufindenden Keime sind überdurchschnittlich starke Biofilmbildner. Dadurch wird der therapeutische Zugang bei einer manifesten Infektion massiv erschwert und Chronifizierung bzw. häufige Rückfälle im Infektionsgeschehen sind die Folge. Rezente Untersuchungen bestätigen, dass eine Wiederinfektion häufig durch den Partner erfolgt. Eine Partnerbehandlung ist daher immer angezeigt. Bei Männern finden sich die Keimreservoirs zumeist in der Umschlagfalte der Vorhaut und/oder im ersten Drittel der Urethra.

Bakterielle Dysbiosen und Infektionen lassen sich durch eine Arzneimittelkombination bestehend aus einem antibakteriellen Arzneistoff mit einem antiadhäsiven Wirkstoff effizient therapieren. Durch die Adhäsionshemmung wird der Biofilm aufgebrochen und vom Epithel des Wirtes abgelöst. Damit werden gleichzeitig alle darin enthaltenen Keime freigesetzt und der direkten antibiotischen Behandlung wieder zugänglich gemacht.

Eine besonders beanspruchte Arzneistoffklasse unter den antiadhäsiven Wirkstoffen sind die nicht steroidalen anti-inflammatorischen Arzneistoffe (NSAIDs), Diese weisen zusätzlich zum antiadhäsiven Effekt den einer sofortigen Schmerzhemmung auf, was in Anbetracht der häufig mit Schmerzen verbundenen Vaginalinfektionen ein weiterer besonderer Vorteil ist. Entscheidend für das Aufbrechen des Biofilms ist die Anwesenheit des NSAID in den beschriebenen Konzentrationen und Bedingungen in einer Emulsion in der beschriebenen Zusammensetzung und in den beschriebenen Mengenverhältnissen. Im Sinne der Erfindung besonders geeignete NSAIDs sind Diclofenac, Bufexamac, Ibuprofen, Dexibuprofen, Flurbiprofen, Ketoprofen, Piroxicam, Meloxicam, Flufenaminsäure, Mefenaminsäure, Indometacin oder Naproxen.

**Tab: A - Beispiele für übliche und bevorzugte erfindungsgemäße Konzentrationen**

| Wirkstoff | Einzeldosis | Tageshöchstdosis | Beispiel | Einzeldosis | Tageshöchstdosis | Beispiel | **Erfindungsgemäße Konzentration** |
|---|---|---|---|---|---|---|---|
| | oral/i.v. | | | topisch | | | |
| Diclofenac Natrium | 50 - 75 mg | 150 - 225 mg | Voltaren 50 mg Tabletten | | | Voltadol Schmerzgel 1%, 10 mg/g | **0,2 - 0,5 Gew%** |
| | | | 75 mg Amp. | 40 - 80 mg | 240 mg | 2%, 20 mg/g | |
| Indometacin | 25 - 75 mg | 50 - 150 mg | Indocid 25 mg | 20-40 mg | 40-80 mg | IndometGel | **0,1 - 0,4 Gew.%** |
| | | | 75 mg ret. | | | 1%, 10 mg/g | |
| Naproxen | 250 - 500 mg | 1000 mg | Naproxen FT 250/500 mg | | | | |
| | | | Naproxen Susp. 50 mg/ml | | | | **0,5 - 2,5 Gew%** |
| Ibuprofen | 200 - 800 mg | 2400 mg | Ibuprofen FT 200/400/800 mg | | | Ibutop Creme/ Gel | |
| | | | | 100 -250 mg | 1000 mg | 5%, 5g/100 g | **0,5-2,5 Gew.%** |

Die Konzentrationseinschränkung It. Tabelle 1 gilt für die Anwendung auf offenen Wunden (möglich im Fall dermaler Pilzinfektionen) und auf Schleimhäuten. Für die Anwendung auf weniger stark entzündeter Haut ist eine NSAID-Konzentration bis maximal zur üblichen topischen Menge möglich.

Unter den bakteriellen Mikroorganismen, welche sich in den vaginalen Biofilmen finden, spielen typische meist fakultativ anaerobe Darmkeime, wie Enterobacter,. E. coli, Klebsiella pneumoniae und Enterokokken, aber auch Ureaplasmen und Mykoplasmen, sowie Gardnerella vaginalis, Prevotella spp. und Mobiluncus eine besondere Rolle. Bevorzugte Antibiotika zur Behandlung dieser Keime sind Phosphomycin, Clindamycin, Metronidazol, Nitrofurantoin, Nitrofurazon, Nitrofurantoin, Nifuratel, Nifuroxacin, Nitroxolin, Trimethoprim, Sulfadiazin, Cotrimoxazol.

Da Antiseptika bei höheren Konzentrationen ebenfalls eine antibakterielle Wirkung ausüben, sind auch diese alleine oder gemeinsam mit einem Antibiotikum zur Kombination mit einem antiadhäsiven Wirkstoff gemäß der Erfindung geeignet. Bevorzugte Antiseptika sind quartäre Ammoniumsalze, wie Benzalkoniumchlorid, und Dequaliniumchlorid, sowie Phenoxyethanol und Propylenglykol. Die Zusammensetzung der erfindungsgemäßen Arzneistoffkombinationen ist in den beschriebenen Kombinationen für die Behandlung auch komplexer chronischer Scheidenentzündungen ganz besonders geeignet.

In speziellen Applikationen ist es zweckmäßig, der Emulsion einen Geruchsstoff hinzuzufügen. Der Zusatz eines Terpens zur Emulsion, vorzugsweise von Farnesol, welches zusätzlich einen Biofilmhemmenden Effekt hat, stellt daher einen bevorzugten Gegenstand der Erfindung dar.

Emulsionen gemäß der vorliegenden Erfindung sind auch zur Behandlung von urogenitalen Schleimhauterkrankungen des Mannes an der Glans penis und in der Urethra geeignet.

Blasenentzündungen sind weit verbreitet. Sie werden meist durch bakterielle Infektion verursacht. Die Blase ist für eine topische Behandlung schlecht zugänglich. Da sich allerdings die Harnröhre der Frau im vorderen Scheideneingang befindet, verläuft der häufigste Infektionsweg von der Scheide aus in die Harnröhre hinein. Außerdem werden in der urologischen und gynäkologischen Praxis auch häufig Beschwerdebilder gesehen, welche sich auf eine isolierte Entzündung der Harnröhre zurückführen lassen. Die Vagina wird somit zum primären Keimreservoir für Harnröhren- und Blasenentzündungen. Wenn auch eine Unterstützung der antibiotischen Behandlung von akuten Blasenentzündungen durch die orale Gabe von adhäsionshemmenden Pflanzenextrakten, welche sich an der Blasenwand festsetzen, erreicht werden kann, so ist dennoch die Sanierung der meist ebenso von der Infektion betroffenen Scheidenflora die Grundvoraussetzung für eine nachhaltige Heilung. Diese kann mit den erfindungsgemäßen Arzneimitteln erreicht werden. Aus diesem Grund sind Arzneimittel gemäß der vorliegenden Erfindung effiziente Therapeutika gegen Blasenentzündung, sowohl alleine, als auch in Kombination mit nur in der Blase wirkenden Arzneimitteln.

Abgesehen von rein bakteriellen Infektionen kommt es häufig auch zu Mischinfektionen mit Pilzen (überwiegend Hefen der Gattung Candida). In der WO 2007/131253 A2 werden Arzneimittelkombinationen zur topischen Behandlung von Pilzinfektionen beansprucht, deren besondere Wirkung darauf beruht, dass zusätzlich zum antimykotischen Wirkstoff ein weiterer Wirkstoff hinzukommt, der die Adhäsion des Pilzes am Epithel unterbindet. Manche der genannten Mischinfektionen können durch die in der WO 2007/131253 A2 beanspruchten Kombinationen therapiert werden. In schweren Fällen von Mischinfektionen ist es allerdings zweckmäßig bzw. notwendig, sich nicht auf die Behandlung der Pilzinfektion zu beschränken, sondern das Arzneimittel durch einen antibakteriellen Wirkstoff in einer Tripelkombination zu ergänzen.

Der antimykotische Wirkstoff ist vorzugsweise ein Arzneistoff aus der Gruppe Nystatin, Ciclopirox oder Ciclopiroxolamin, oder einer aus der Gruppe der Azole (Imidazole, Triazole, Tetraazole) wie Clotrimazol, Fluconazol, Miconazol, Itraconazol, Tioconazol, Voriconazol, Bifonazol, Econazol, Isoconazol, Fenticonazol, Sertaconazol, Ketoconazol, Posaconazol, Quilseconazol, VT-1161, SCY-078.

Arzneimittel gemäß der vorliegenden Erfindung wurden zunächst zur Therapie von Vaginosen und Blasenentzündungen entwickelt, sie können aber auch gleichermaßen zur Behandlung bakterieller Hautkrankheiten verwendet werden.

Sowohl die Akne als auch der genetisch bedingte Haarausfall werden mit dem männlichen Sexualhormon Testosteron in Zusammenhang gebracht. Tatsächlich besteht der Beitrag dieses Hormons am pathogenetischen Geschehen primär in der Aktivierung von Talgdrüsen in der Haut und in den Haarbälgen. Der angesammelte Talg ist in der Folge ein ideales Substrat sowohl für eine bakterielle Infektion, vor allem mit Propionibakterien oder mit den Pilzen Candida und Malassezia. Während Malassezia vorwiegend mit der Pityriasis versicolor in Zusammenhang gebracht wird, steht bei der Akne Propionibacterium acnis im Vordergrund. Beim Haarausfall sind beide beteiligt. Die große Menge an Talg, welche sich in den Haarfollikeln ansammelt, ist ein ideales Substrat für die Ausbildung eines Biofilms. Grundsätzlich gelten für diesen Biofilm dieselben Kriterien wie für Biofilme, welche sich in der Scheide bilden. Auch in diesem Fall muss zunächst der Biofilm aufgebrochen und seine Anhaftung am Epithel aufgelöst werden, damit in der Folge das Antiinfektivum seine Wirkung ausüben kann. Demgemäß sind die Emulsionen gemäß der vorliegenden Erfindung auch zur Behandlung von Hautkrankheiten, vor allem von Pityriasis versicolor, Akne und Haarausfall sehr gut geeignet. Da die dermalen Anwendungen ein wesentlich größeres und deutlich weniger fest umschriebenes Areal betreffen können, als Scheideninfektionen, können in diesen Fällen spezielle Emulsionen bevorzugt zur Anwendung kommen. Insbesondere sind zur Behandlung des Haarausfalls Shampoos und zur Behandlung der Akne Akne-Cremes, Akne-Sticks oder Akne-Lösungen geeignet.

Die Erfindung wird anhand der nachfolgenden Beispiele näher erläutert, auf die sie jedoch nicht eingeschränkt ist.

### Beispiele:

### Emulsionen welche Phenoxyethanol als antibakterielles Agens enthalten

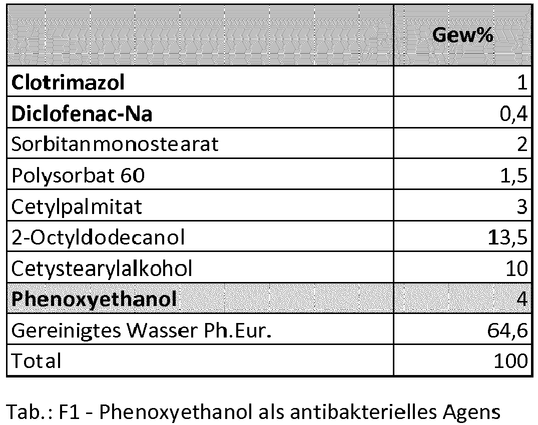

**Fallbeispiel:** 24jährige Patientin (EH), seit 3 Jahren fast ständige Beschwerden durch Pilzinfektionen, im GV seit Jahren kaum möglich. Seit 1 Woche extreme Beschwerden. Brennen und Jucken im Introitus.
Gyn.U: V+V mass. gerötet, mass. rahmig-grünl. SI, Sekretabstrich nativ: massenhaft an den Epithelzellen und an massenhaft Pilzhyphen haftende Leukozyten, Mischflora, wenig Lactobac., schmutziger Hintergrund - lytische Zellen, RG IIb
Therapie: F1 über 2 Wochen, 0-0-1 Hb vaginal appliziert, anschließend F1 bei Bedarf.
Ko. nach 3 Jahren: seit letzter Therapie beschwerdefrei;
Gyn.U.: SH bland, normale Scheidenflora, RG I

### Beispiel: Emulsionen, welche Dequaliniumchlorid enthalten

**Tab: F2 - Dequaliniumchlorid als antibakterielles Agens**

| Zusammensetzung der Emulsion | Gew% |
|---|---|
| **Clotrimazol** | 1 |
| **Diclofenac Na** | 0,3 |
| Sorbitanmonostearat | 2 |
| Polysorbat 60 | 1,5 |
| Cetylpalmitat | 3 |
| 2-Octyldodecanol | 13,5 |
| Cetostearylalkohol | 10 |
| Phenoxyethanol | 1 |
| **Dequaliniumchlorid** | 0,4 |
| Gereinigtes Wasser Ph.Eu. | 67,3 |
| Gesamt | 100 |

**Fallbeispiel:** 40jährige Patientin (AKS), seit Jahren fast monatlich Beschwerden durch Pilzinfektionen, jeweils über ca. 1 Woche bis 10 Tage, GV seit Jahren kaum möglich. Seit 1 Woche zusätzlich Vaginalsekret dünnflüssig und übelriechend. Brennen und Jucken im Introitus.
Gyn.U: SH stark gerötet, Sekret dünnflüssig, leicht grünlich. Sekretabstrich nativ: reichl. Biofilmplaques auf dicken Pilzhyphen, Leuko+++, kaum Lactobac. (RG IIb).
Therapie: F2 über 1 Woche, 0-0-1 Hb vaginal appliziert.
Ko. nach 2 Jahren: seit 2 Jahren beschwerdefrei, nur gelegentlich sehr leichte Pilzinfektion, mit restl.
F2 behandelt; Gyn.U.: SH bland, RG I

**Tab.: F3 - Dequaliniumchlorid als antibakterielles Agens**

| Zusammensetzung der Emulsion | Gew% |
|---|---|
| **Clotrimazol** | 1,00 |
| **Diclofenac Na** | 0,20 |
| Sorbitanmonostearat | 2,00 |
| Polysorbat 60 | 1,50 |
| Cetylpalmitat | 3,00 |
| 2-Octyldodecanol | 13,50 |
| Cetostearylalkohol | 10,00 |
| Propylenglykol | 7,00 |
| **Dequaliniumchlorid** | 0,40 |
| Gereinigtes Wasser Ph.Eu. | 61,40 |
| Gesamt | 100,00 |

**Fallbeispiel:** 31jährige Patientin (FJ), seit ca. 3 Jahren fast monatlich prämenstruelle Beschwerden, jeweils über ca. 1 Woche bis 10 Tage, durch rezidivierende Pilzinfektionen. Seit 1 Woche zusätzlich Vaginalsekret dünnflüssig und übelriechend. Brennen und Jucken im Introitus.
Gyn.U: SH leicht gerötet, Sekret dünnflüssig, Odor. Sekretabstrich nativ: bakterielle Vaginose (RG III), zusätzlich Pilzhyphen, reichl. Leukozyten.
Therapie: F3 über 1 Woche, 0-0-1 Hb vaginal appliziert.
Kontrolluntersuchung nach 4 Monaten: subj. seit Therapie keine Beschwerden mehr. Gyn.U: Schleimhaut bland, Sekretabstrich nativ: normale Scheidenflora (RG I)

### Beispiel: Emulsionen, welche Clindamycin enthalten

**Tab: F4**

| Zusammensetzung der Emulsion | Gew% |
|---|---|
| **Clindamycin** | **2** |
| **Diclofenac Na** | **0,3** |
| Sorbitanmonostearat | 2 |
| Polysorbat 60 | 1,5 |
| Cetylpalmitat | 3 |
| 2-Octyldodecanol | 13,5 |
| Cetostearylalkohol | 10 |
| Propylenglykol | 7 |
| Phenoxyethanol | 1 |
| Gereinigtes Wasser Ph.Eu. | 59,7 |
| Gesamt | 100 |

**Fallbeispiel:** 31jährige Patientin (EK), seit Monaten therapieresistente. bakterielle Vaginose, übelriechender Ausfluss, V.a. postmenstruell. Im mikrobiol Abstrichbefund reichl. Gardnerella und Prevotella.
Gyn.U: SH stark gerötet, Vaginalsekret dünnflüssig und übelriechend. Sekretabstrich nativ: reichl. Clue cells, Leuko+++. (RG III).
Therapie: F4 über 1 Woche, 0-0-1 vaginal appliziert., anschließend Vagilact.
Ko. nach 2 Jahren: seit 1 Jahr beschwerdefrei, einmal sehr leichte Pilzinfektion; Gyn.U.: SH bland, RG I

### Beispiel: Emulsionen, welche Phosphomycin-Trometamol enthalten

**Tab: F5**

| Zusammensetzung der Emulsion | Gew% |
|---|---|
| **Phosphomycin-Trometamol** | 2 |
| **Diclofenac-Na** | 0,4 |
| Sorbitanmonostearat | 2 |
| Polysorbat 60 | 1,5 |
| Cetylpalmitat | 3 |
| 2-Octyldodecanol | 13,5 |
| Cetystearylalkohol | 10 |
| Phenoxyethanol | 1 |
| Gereinigtes Wasser Ph.Eur. | 66,6 |
| Total | 100 |

**Fallbeispiel:** 69jährige Patientin (ET), seit ca. 2 Jahren rezidivierende Harnwegsinfekte, immer wieder mit Ciproxin behandelt. Seit 2 Wochen wieder verstärkter Harndrang und Brennen im Introitus. Gyn.U: DS über Blase; SH atroph, blutet auf Kontakt, MM atroph, mit Cervixbrush eröffnet, Sekret: Atrophie, aerobe Mischflora (RGIII), reichl. Leukozyten.
Therapie: F5 über 1 Woche, 0-0-1 Hb vaginal appliziert, zusätzlich Ovestin.
Kontrolluntersuchung nach 2 Wochen: subj. seit Therapie keine Beschwerden mehr.
Gyn.U: Schleimhaut bland, kein DS über Blase und Urethra; Sekretabstrich nativ: beginnend normale Scheidenflora (RG I), keine Leukozyten.

**Tab.: F6**

| Zusammensetzung der Emulsion | Gew% |
|---|---|
| **Phosphomycin-Trometamol** | 2 |
| **Clotrimazol** | 1 |
| **Diclofenac Na** | 0,3 |
| Sorbitanmonostearat | 2 |
| Polysorbat 60 | 1,5 |
| Cetylpalmitat | 3 |
| 2-Octyldodecanol | 13,5 |
| Cetostearylalkohol | 10 |
| Propylenglykol | 7 |
| Phenoxyethanol | 1 |
| Gereinigtes Wasser Ph.Eu. | 58,7 |
| Gesamt | 100 |

**Fallbeispiel:** 36jährige Patientin (CS), vor 2 Jahren erstmals HWI nach Thailandurlaub mit Makrohämaturie und lang andauerndem Brennen, seit 2 Monaten wieder UB-Schmerzen und HWI (wieder nach Thailandurlaub), immer wieder Brennen v.a. nach dem Urinieren, seit 2 Wochen wieder verstärkter Harndrang, nach AB-Behandlung (1 Woche) keine Besserung der Blasenbeschwerden aber nun zusätzlich Jucken und Brennen im Introitus.
Gyn.U: DS über Blase; Vaginal-SH vom Aspekt her eher unauffällig, ausgeprägte Zervicitis mit ca. 1 cm messender blutiger Erosion um den MM, Sekret Nativpräparat: RG III, massenhaft Leukozyten, diese mit Bakterien belegt, wenig Lactobacillen, Hyphen ++. Mikrobiol Abstrich - Sekretkultur: massenhaft E.coli.
Therapie: F6 über 10 Tage, 0-0-1 vaginal appliziert, zusätzlich Monuril 2 x 1 Btl/Woche. Kontrolluntersuchung nach 4 Wochen: subj. seit Therapie keine Beschwerden mehr.
Gyn.U: Schleimhaut bland, kein DS über Blase und Urethra; Sekretabstrich nativ: normale Scheidenflora (RG I), keine Leukozyten.

**Tab: F7**

| Zusammensetzung der Emulsion | Gew% |
|---|---|
| **Phosphomycin-Trometamol** | **2** |
| **Diclofenac Na** | **0,3** |
| Sorbitanmonostearat | 2 |
| Polysorbat 60 | 1,5 |
| Cetylpalmitat | 3 |
| 2-Octyldodecanol | 13,5 |
| Cetostearylalkohol | 10 |
| **Propylenglykol** | **7** |
| Phenoxyethanol | 1 |
| Gereinigtes Wasser Ph.Eu. | 59,7 |
| Gesamt | 100 |

### Beispiel: Shampoo zur Behandlung von pilzbedingtem Haarausfall:

**Tab: F8**

| Zusammensetzung der Emulsion | Gew% |
|---|---|
| Ketoconazol | 2 |
| Diclofenac Na | 0,3 |
| Ammoniumlaurylsulfat | 15,00 |
| Lauramid | 4,00 |
| Natriumchlorid | 1,00 |
| Farnesol | 1 |
| Dinatrium EDTA | 0,2 |
| Methylparaben | 0,08 |
| Propylparaben | 0,05 |
| Gereinigtes Wasser Ph.Eu. | 76,37 |
| 100 | |

Strukturformel von SCY-078 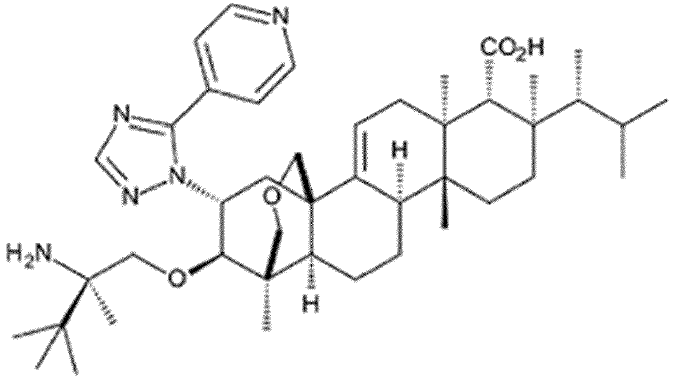
Strukturformel von VT-1161

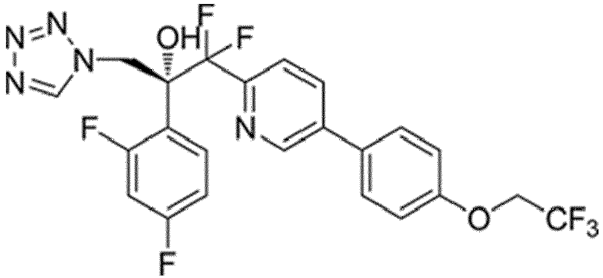

Demgemäß betrifft die Erfindung die nachfolgenden, bevorzugten Ausführungsformen::
1. Eine Emulsion, dadurch gekennzeichnet, dass ein antibakterieller Wirkstoff und ein antiadhäsiver Wirkstoff, vorzugsweise ein NSAID, kombiniert eingesetzt werden.
2. Eine Emulsion gemäß Ausführungsform 1, welche zusätzlich einen antimykotischen Wirkstoff enthält.
3. Eine Emulsion gemäß Ausführungsform 1 oder 2, vorzugsweise in Form eines Salbe oder einer Creme, mit einer wässerigen Phase und einer Ölphase, enthaltend einen antibakteriellen Wirkstoff und ein NSAID, dadurch gekennzeichnet, dass (a) das NSAID in der wässerigen Phase in einem Konzentrationsbereich vorliegt, welcher für die Anwendung auf stark entzündeter Haut und auf Schleimhäuten der Hälfte bis einem Zehntel der für diese Wirkstoffe üblichen Konzentration entspricht, dass (b) das Gewichtsverhältnis der Wasser zur Ölphase in dieser Emulsion zwischen den Werten 2,1 und 2,9 liegt, und dass (c) der pH Wert der Emulsion nicht unter dem Wert 6,5 und nicht über 8,5 vorzugsweise im Bereich 7,0 bis 8 liegt, vorzugsweise zur Behandlung von urogenitalen Infektionskrankheiten, insbesondere zur Anwendung in der topischen Behandlung von vaginalen Infektionen und von Blasenentzündungen der Frau sowie zur lokalen Partnerbehandlung (Glans penis, Anfangsdrittel der Urethra).
4. Eine Emulsion gemäß Ausführungsform 1 oder 2, vorzugsweise in Form eines Salbe, einer Creme, eines Shampoos, einer Lösung oder eines Sticks, mit einer wässerigen Phase und einer Ölphase, enthaltend einen antibakteriellen Wirkstoff und ein NSAID, dadurch gekennzeichnet, dass (a) das NSAID in der wässerigen Phase in einem Konzentrationsbereich vorliegt, welcher bei der kutanen Applikation höchstens der üblichen Konzentration, bei der Anwendung auf Schleimhäuten der Hälfte bis einem Zehntel der für diese Wirkstoffe üblichen Konzentration entspricht und dass (b) der pH Wert der Emulsion nicht unter dem Wert 5,5 und nicht über 8,5 liegt, vorzugsweise zur topischen Behandlung von dermalen Infektionen, insbesondere zur Anwendung in der topischen Behandlung von Akne, Haarausfall, Pytiriasis versicolor und atopischer Dermatitis.
5. Eine Emulsion gemäß einer der Ausführungsformen 1 bis 4, dadurch gekennzeichnet, dass das NSAID Diclofenac, Bufexamac, Ibuprofen, Dexibuprofen, Flurbiprofen, Ketoprofen, Piroxicam, Meloxicam, Flufenaminsäure, Mefenaminsäure, Indometacin oder Naproxen ist.
6. Eine Emulsion gemäß einer der Ausführungsformen 1 bis 5 dadurch gekennzeichnet, dass das Antibiotikum Phosphomycin, Clindamycin, Metronidazol, Nitrofurantoin, Nitrofurazon, Nitrofurantoin, Nifuratel, Nifuroxacin, Nitroxolin, Trimethoprim, Sulfadiazin, oder Cotrimoxazol ist.
7. Eine Emulsion gemäß einer der Ausführungsformen 1 bis 6 dadurch gekennzeichnet, dass der antimikrobielle Effekt durch ein Antiseptikum erreicht wird.
8. Eine Emulsion gemäß Ausführungsform 7 dadurch gekennzeichnet, dass das Antiseptikum Benzalkoniumchlorid, Dequaliniumchlorid, Phenoxyethanol oder Propylenglykol ist.
9. Eine Emulsion gemäß einer der Ausführungsformen 2 bis 8, dadurch gekennzeichnet, dass der antimykotische Wirkstoff Nystatin, Ciclopirox oder Ciclopiroxolamin, oder ein Antimykotikum aus der Gruppe der Azole, vorzugsweise Clotrimazol, Fluconazol, Miconazol, Itraconazol, Tioconazol, Voriconazol, Bifonazol, Econazol, Isoconazol, Fenticonazol, Sertaconazol, Ketoconazol, Posaconazol, Quilseconazol, VT-1161 oder SCY-078, ist.
10. Eine Emulsion gemäß einer der Ausführungsformen 1 bis 9, dadurch gekennzeichnet, dass das NSAID Diclofenac ist und dieses in einem Konzentrationsbereich von 0,2 - 0,4 Gewichtprozent der Emulsion enthalten ist.
11. Eine Emulsion gemäß einer der Ausführungsformen 1 bis 10 zur Anwendung in der topischen Behandlung von dermalen und urogenitalen Infektionskrankheiten, insbesondere zur Anwendung in der topischen Behandlung von urogenitalen bakteriellen Infektionen.
12. Emulsion gemäß einer der Ausführungsformen 1 bis 10 zur Anwendung in der Behandlung von Infektionskrankheiten, insbesondere von bakteriellen urogenitalen Infektionen der Frau und von asymptomatischen und symptomatischen Dysbiosen der Glans penis und der männlichen Urethra.
13. Emulsion gemäß einer der Ausführungsformen 1 bis 10 zur Anwendung in der Behandlung von Infektionskrankheiten, insbesondere von vaginalen Mischinfektionen durch Candida albicans und Bakterien wie Enterobacter, E.coli, Klebsiella pneumoniae, Gardnerella vaginalis, Prevotella spp.
14. Emulsion gemäß einer der Ausführungsformen 1 bis 10 zur Anwendung in der Behandlung asymptomatischer und symptomatischer bakterieller Vaginosen und von asymptomatischen und symptomatischen Dysbiosen der Glans penis und der männlichen Urethra.
15. Emulsion gemäß einer der Ausführungsformen 1 bis 10 zur Anwendung in der Behandlung von Akne.
16. Emulsion gemäß Ausführungsform 15 dadurch gekennzeichnet, dass die Emulsion zu einem Akne-Stick oder einer Anke-Lösung verarbeitet ist.
17. Emulsion gemäß einer der Ausführungsformen 1 bis 10 zur Anwendung in der Behandlung dermaler Pilzinfektionen, vorzugsweise von Candidamykosen und Malasseziamykosen.
18. Emulsion vorzugsweise Shampoos gemäß einer der Ausführungsformen 1 bis 10 zur Behandlung von Haarausfall.
19. Emulsion gemäß einer der Ausführungsformen 1 bis 18, dadurch gekennzeichnet, dass sie weiters einen Geruchsstoff enthält, vorzugsweise ein Terpen, insbesondere Farnesol.
20. Ein Verfahren zur Herstellung einer Emulsion gemäß einer der Ausführungsformen 1 bis 19, dadurch gekennzeichnet, dass bei der Herstellung der Emulsion das NSAID über die wässrige Phase eingebracht wird,
21. Ein Verfahren zur Herstellung einer Emulsion gemäß einer der Ausführungsformen 1 bis 19, dadurch gekennzeichnet, dass das NSAID als feinkristallines oder mikronisiertes Salz in die das Antimykotikum enthaltene Emulsion eingebracht wird.

## Patentansprüche

1. Eine Emulsion, **dadurch gekennzeichnet, dass** ein antibakterieller Wirkstoff und ein antiadhäsiver Wirkstoff, vorzugsweise ein NSAID, kombiniert eingesetzt werden.

2. Eine Emulsion gemäß Anspruch 1, welche zusätzlich einen antimykotischen Wirkstoff enthält.

3. Eine Emulsion gemäß Anspruch 1 oder 2, vorzugsweise in Form eines Salbe oder einer Creme, mit einer wässerigen Phase und einer Ölphase, enthaltend einen antibakteriellen Wirkstoff und ein NSAID, **dadurch gekennzeichnet, dass** (a) das NSAID in der wässerigen Phase in einem Konzentrationsbereich vorliegt, welcher für die Anwendung auf stark entzündeter Haut und auf Schleimhäuten der Hälfte bis einem Zehntel der für diese Wirkstoffe üblichen Konzentration entspricht, dass (b) das Gewichtsverhältnis der Wasser zur Ölphase in dieser Emulsion zwischen den Werten 2,1 und 2,9 liegt, und dass (c) der pH Wert der Emulsion nicht unter dem Wert 6,5 und nicht über 8,5 vorzugsweise im Bereich 7,0 bis 8 liegt, vorzugsweise zur Behandlung von urogenitalen Infektionskrankheiten, insbesondere zur Anwendung in der topischen Behandlung von vaginalen Infektionen und von Blasenentzündungen der Frau sowie zur lokalen Partnerbehandlung (Glans penis, Anfangsdrittel der Urethra).

4. Eine Emulsion gemäß Anspruch 1 oder 2, vorzugsweise in Form eines Salbe, einer Creme, eines Shampoos, einer Lösung oder eines Sticks, mit einer wässerigen Phase und einer Ölphase, enthaltend einen antibakteriellen Wirkstoff und ein NSAID, **dadurch gekennzeichnet, dass** (a) das NSAID in der wässerigen Phase in einem Konzentrationsbereich vorliegt, welcher bei der kutanen Applikation höchstens der üblichen Konzentration, bei der Anwendung auf Schleimhäuten der Hälfte bis einem Zehntel der für diese Wirkstoffe üblichen Konzentration entspricht und dass (b) der pH Wert der Emulsion nicht unter dem Wert 5,5 und nicht über 8,5 liegt, vorzugsweise zur topischen Behandlung von dermalen Infektionen, insbesondere zur Anwendung in der topischen Behandlung von Akne, Haarausfall, Pytiriasis versicolor und atopischer Dermatitis.

5. Eine Emulsion gemäß einem der Ansprüche 1 bis 4, **dadurch gekennzeichnet, dass** das NSAID Diclofenac, Bufexamac, Ibuprofen, Dexibuprofen, Flurbiprofen, Ketoprofen, Piroxicam, Meloxicam, Flufenaminsäure, Mefenaminsäure, Indometacin oder Naproxen ist; und/oder das Antibiotikum Phosphomycin, Clindamycin, Metronidazol, Nitrofurantoin, Nitrofurazon, Nitrofurantoin, Nifuratel, Nifuroxacin, Nitroxolin, Trimethoprim, Sulfadiazin, oder Cotrimoxazol ist.

6. Eine Emulsion gemäß einem der Ansprüche 1 bis 5 **dadurch gekennzeichnet, dass** der antimikrobielle Effekt durch ein Antiseptikum erreicht wird, wobei das Antiseptikum vorzugsweise Benzalkoniumchlorid, Dequaliniumchlorid, Phenoxyethanol oder Propylenglykol ist.

7. Eine Emulsion gemäß einem der Ansprüche 2 bis 6, **dadurch gekennzeichnet, dass** der antimykotische Wirkstoff Nystatin, Ciclopirox oder Ciclopiroxolamin, oder ein Antimykotikum aus der Gruppe der Azole, vorzugsweise Clotrimazol, Fluconazol, Miconazol, Itraconazol, Tioconazol, Voriconazol, Bifonazol, Econazol, Isoconazol, Fenticonazol, Sertaconazol, Ketoconazol, Posaconazol, Quilseconazol, VT-1161 oder SCY-078, ist.

8. Eine Emulsion gemäß einem der Ansprüche 1 bis 7, **dadurch gekennzeichnet, dass** das NSAID Diclofenac ist und dieses in einem Konzentrationsbereich von 0,2 - 0,4 Gewichtprozent der Emulsion enthalten ist.

9. Eine Emulsion gemäß einem der Ansprüche 1 bis 8 zur Anwendung in der topischen Behandlung von dermalen und urogenitalen Infektionskrankheiten, insbesondere zur Anwendung in der topischen Behandlung von urogenitalen bakteriellen Infektionen.

10. Emulsion gemäß einem der Ansprüche 1 bis 8 zur Anwendung in der Behandlung von Infektionskrankheiten, insbesondere von bakteriellen urogenitalen Infektionen der Frau und von asymptomatischen und symptomatischen Dysbiosen der Glans penis und der männlichen Urethra; zur Anwendung in der Behandlung von Infektionskrankheiten, insbesondere von vaginalen Mischinfektionen durch Candida albicans und Bakterien wie Enterobacter, E.coli, Klebsiella pneumoniae, Gardnerella vaginalis, Prevotella spp.; zur Anwendung in der Behandlung asymptomatischer und symptomatischer bakterieller Vaginosen und von asymptomatischen und symptomatischen Dysbiosen der Glans penis und der männlichen Urethra; zur Anwendung in der Behandlung dermaler Pilzinfektionen, vorzugsweise von Candidamykosen und Malasseziamykosen; und/oder zur Anwendung in der Behandlung von Akne.

11. Emulsion gemäß Anspruch 10 **dadurch gekennzeichnet, dass** die Emulsion zu einem Akne-Stick oder einer Anke-Lösung verarbeitet ist.

12. Emulsion vorzugsweise Shampoos gemäß einem der Ansprüche 1 bis 10 zur Behandlung von Haarausfall.

13. Emulsion gemäß einem der Ansprüche 1 bis 12, **dadurch gekennzeichnet, dass** sie weiters einen Geruchsstoff enthält, vorzugsweise ein Terpen, insbesondere Farnesol.

14. Ein Verfahren zur Herstellung einer Emulsion gemäß einem der Ansprüche 1 bis 13, **dadurch gekennzeichnet, dass** bei der Herstellung der Emulsion das NSAID über die wässrige Phase eingebracht wird,

15. Ein Verfahren zur Herstellung einer Emulsion gemäß einem der Ansprüche 1 bis 13, **dadurch gekennzeichnet, dass** das NSAID als feinkristallines oder mikronisiertes Salz in die das Antimykotikum enthaltene Emulsion eingebracht wird.
